(19) Europäisches Patentamt
European Patent Office
Office européen des brevets

(11) Publication number: **0 104 601 B1**

(12) **EUROPEAN PATENT SPECIFICATION**

(45) Date of publication of patent specification: **17.12.86**

(51) Int. Cl.[4]: **C 07 C 85/06**, C 07 C 87/60

(21) Application number: **83109388.5**

(22) Date of filing: **21.09.83**

(54) **Method for producing tertiary amines from highly hindered aromatic secondary amines and carbonates.**

(30) Priority: **23.09.82 US 422241**

(43) Date of publication of application:
**04.04.84 Bulletin 84/14**

(45) Publication of the grant of the patent:
**17.12.86 Bulletin 86/51**

(84) Designated Contracting States:
**CH DE FR GB LI**

(56) References cited:
**FR-A-2 348 910**
**GB-A-1 019 912**
**GB-A-2 044 261**
**US-A-3 317 605**

(73) Proprietor: **PPG INDUSTRIES, INC.**
**One PPG Place**
**Pittsburgh Pennsylvania 15272 (US)**

(72) Inventor: **Sienkowski, Kenneth James**
**1531 Brunette Drive**
**Downers Grove, IL 60516 (US)**
Inventor: **Thompson, Ralph Brewster**
**Adams Road**
**Oakbrook, IL 60521 (US)**

(74) Representative: **Hann, Michael, Dr. et al**
**Patentanwälte Dr. Michael Hann Dr. H.-G. Sternagel Sander Aue 30**
**D-5060 Bergisch Gladbach 2 (DE)**

**EP 0 104 601 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention refers to N,N-bis(2,4,6-tribromophenyl)methylamine and a method for its preparation.

It is well known to use brominated aromatic compounds as flame retardant additives for polymers. Usually, highly brominated compounds show better flame retardant properties than lower brominated ones. So, N,N-bis(2,4,6-dibromophenyl) lower alkylamines which have been disclosed by Weinstock in US—A—3 317 605 can be expected to be less effective flame retardants than N,N-bis(2,4,6-tribromophenyl) lower alkylamines.

N,N-bis(2,4,6-dibromophenyl)methylamine resists further bromination, that means N,N-bis(2,4,6-tribromophenyl)methylamine cannot be prepared in high yield and purity by conventional bromination methods.

The alkylation of many aromatic secondary amines is customarily accomplished by reaction of the amine with a dialkyl sulfate.

Dimethyl sulfate is typical of the dialkyl sulfates employed for this purpose. Alkylation of many aromatic secondary amines may also be effected using a dialkyl carbonate as the alkylation agent, such as is disclosed in BE—A—882,135 and FR—A—2 348 910. When the amino hydrogen atom of the amine to be alkylated is substantially sterically unhindered, either of these methods may in most cases be used to achieve alkylation.

As it is disclosed in GB—A—1 019 912 aromatic carbocyclic or heterocyclic secondary amines are subject to an aminoalkylation by treating of such amines which could also be sterically hindered with dialkyl carbonates carrying at least one tertiary substituted amino group in position 2 or 3 of the hydrocarbon chain. Furthermore, it was illustrated in Example XIII of the same application that by heating of a mixture containing 2-chlorophenothiazine, 3-dimethylamino-1-propanol and diethyl carbonate in presence of anhydrous potassium carbonate as catalyst up to 200°C for several hours 2-chloro-10(3$^1$-dimethylaminopropyl)phenothiazine is obtained in good yield but no or only slight amounts of 2-chloro-10-ethyl phenothiazine are formed although the reaction mixture contained diethyl carbonate quite in excess. By this knowledge, dialkyl carbonates were expected to be unsuited as alkylating agents at least for sterically hindered aromatic secondary amines.

It has now been found that when the amino hydrogen atom of the aromatic secondary amine is highly sterically hindered, that is to say, the amine is a highly hindered amine, alkylation through use of dialkyl sulfate either produces low yields of the alkylated product or does not produce the product at all.

Object of the invention is to provide the novel compound N,N-bis(2,4,6-tribromophenyl)methylamine and a method for preparing this compound in high yield which overcomes difficulties in alkylating sterically hindered aromatic secondary amines.

According to this invention N,N-bis(2,4,6-tribromophenyl)methylamine is prepared by reacting bis(2,4,6-tribromophenyl)amine with dialkyl carbonate.

In contradiction to prior art it has now been found that many highly hindered aromatic secondary amines, such as bis(2,4,6-tribromophenyl)amine, for which alkylation by dialkyl sulfate is largely ineffectual or inefficient, may be successfully and efficiently alkylated by the use of dialkyl carbonate to produce tertiary amines in greater yield than when dialkyl sulfate is employed. Moreover it has been found that many dialkyl carbonates are useful in converting highly hindered aromatic secondary amines to tertiary amines. Accordingly, the present invention is a method comprising reacting the highly hindered bis(2,4,6-tribromophenyl)amine with dialkyl carbonate of at least one methyl alcohol to produce N,N-bis(2,4,6-tribromophenyl)methylamine.

The organic carbonate of at least one methyl alcohol used in the method is subject to wide variation. A class of particular importance may be represented by the formula

$$H_3CO\underset{\underset{O}{\|}}{C}OR \qquad (I)$$

wherein R is alkyl, aralkyl, (cycloalkyl)alkyl, cycloalkyl. When alkyl is employed, it usually has from 1 to about 20 carbon atoms, often from 1 to about 10 carbon atoms. Lower alkyl having from 1 to about 4 carbon atoms is preferred. Methyl and ethyl are especially preferred. When aralkyl is employed, the aryl portion generally contains from 6 to about 10 carbon atoms and the alkyl portion usually contains from 1 to about 4 carbon atoms; benzyl is preferred. When (cycloalkyl)alkyl is used, the cycloalkyl portion generally contains from about 6 to about 8 carbon atoms and the alkyl portion typically contains from 1 to about 4 carbon atoms; cyclohexylmethyl is preferred. The cycloalkyl typically has from about 6 to about 8 carbon atoms; cyclohexyl is preferred. These groups are usually unsubstituted, although one or more minor substituents which do not render the organic carbonate unsuitable for its intended purpose may be present on any of the groups. Similarly, those groups having one or more rings are usually homocyclic, but one or more hetero atoms may be present so long as they do not preclude tertiary amine formation. The aliphatic groups and the aliphatic portions of hybrid groups such as aralkyl may be straight or branched, but it is preferred they be straight. Only one organic carbonate or a plurality of organic carbonates may be used as desired.

Examples of organic carbonates which may be employed include dimethyl carbonate, ethyl methyl carbonate, propyl methyl carbonate, isopropyl methyl carbonate, butyl methyl carbonate, secondary-butyl methyl carbonate, isobutyl methyl carbonate, tertiary-butyl methyl carbonate, cyclohexyl methyl carbonate, benzyl methyl carbonate. The particularly preferred organic carbonate is dimethyl carbonate.

As highly hindered aromatic secondary amine bis(2,4,6-tribromophenyl)amine can be converted to N,N-bis(2,4,6-tribromophenyl)methylamine according to the invention. The common characteristics of the starting amine which is prepared by formula II is that two aryl groups are attached to the amine nitrogen and the amino hydrogen is highly hindered.

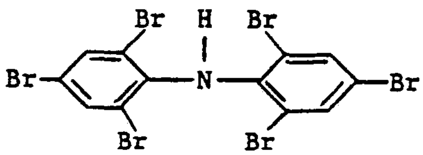

(II)

The reaction of bis(2,4,6-tribromophenyl)amine and dialkyl carbonate is usually conducted in the liquid phase. It may be carried out batchwise, continuously, semibatchwise or semicontinuously. When the dialkyl carbonate is a liquid under the conditions of the reaction, it often acts as a solvent for the bis(2,4,6-tribromophenyl)amine. Typically, but not necessarily, excess organic carbonate is employed and this usually serves to dissolve the bis(2,4,6-tribromophenyl)amine throughout the reaction. In many cases, one or more by-products of the reaction, most notably alcohols, also tend to dissolve the bis(2,4,6-tribromophenyl)amine. Although extrinsic solvent is not ordinarily employed, it may be used when desired or when necessary to dissolve one or more of the reactants. Examples of suitable extrinsic solvents include methanol, ethanol, acetonitrile, benzene, toluene, dioxane, dimethylformamide and chlorinated solvents such as chloroform, methylene chloride, ethylene chloride, carbon tetrachloride and chlorobenzene, only one extrinsic solvent or a plurality of extrinsic solvents may be used as desired. For many reactions, extrinsic solvent need not be introduced, and the reaction may be neat.

When extrinsic solvent is used, the weight ratio of extrinsic solvent to the bis(2,4,6-tribromophenyl)-amine initially present is subject to wide variation. Generally, the amount of solvent should be sufficient to dissolve the reactants at the reaction temperature. The weight ratio of extrinsic solvent, when used, to the bis(2,4,6-tribromophenyl)amine initially present is usually in the range of from about 0.01:1 to about 20:1. From about 0.1:1 to about 5:1 is preferred.

The temperatures at which the reaction is conducted may vary widely, but ordinarily they are in the range of from about 160°C to about 240°C. Preferably the temperatures are in the range of from about 180°C to about 210°C.

The pressures at which the reaction is conducted are similarly susceptible to wide variation. Atmospheric and superatmospheric pressures are generally employed, although lesser pressures may sometimes be used. Generally the pressure is in the range of from about zero to about 5000 kilopascals, gauge, but higher pressures may be used. Preferably the pressure is in the range of from about 500 to about 2000 kilopascals, gauge.

The reaction may be conducted in the presence of catalyst, although in many instances the use of catalyst is not needed. Exemplary catalysts which may be used include nitrogen-containing heterocyclic catalysts such as pyridine, 4-(dimethylamino)pyridine, imidazole, 2,6-lutidine and 2,4,6-collidine. Only a single catalyst or a mixture of catalysts may be used where desired. The preferred catalyst is 4-(dimethylamino)pyridine.

The equivalent ratio of the catalyst, when used, to the bis(2,4,6-tribromophenyl)amine initially present may vary widely but usually it is in the range of from about 0.005:1 to about 0.5:1. It is preferred that the equivalent ratio be in the range of from about 0.01:1 to about 0.2:1.

A preferred embodiment of the invention is the method for methylating bis(2,4,6-tribromophenyl)-amine comprising reacting the amine with dialkyl carbonate in which at least one of the alkyl groups of the dialkyl carbonate is methyl, to produce N,N-bis(2,4,6-tribromophenyl)methylamine. The other alkyl group of the dialkyl carbonate used in this embodiment typically contains from 1 to about 20 carbon atoms, often from 1 to about 10 carbon atoms. Lower alkyl having from 1 to about 4 carbon atoms is preferred. Methyl and ethyl are especially preferred. The methyl and the other alkyl group of the dialkyl carbonate are usually unsubstituted, although the alkyl group may, subject to the conditions of the immediately preceding paragraph, contain minor substituents which do not render the compound unsuitable for its intended purpose. Only one or a mixture of dialkyl carbonates may be used as desired. Dimethyl carbonate is particularly preferred.

Following preparation, the tertiary amine may be recovered from the reaction mixture by any of the various techniques known to the art. Crystallization is one such technique that is frequently employed.

The process of the invention may be employed to produce not only N,N-bis(2,4,6-tribromophenyl)-methylamine but also similar tertiary amines having widely varying uses, as for example, fire retardants for polymers, antioxidants in substrates such as rubber and as intermediates in the process of organic synthesis.

The invention is further described in conjunction with the following examples which are to be considered illustrative rather than limiting.

Example I

A one-liter reactor equipped with an agitator, an automatic temperature controller, a pressure gauge and an electric heating mantle was charged with 50 grams of bis(2,4,6-tribromophenyl)amine and 160 grams of dimethyl carbonate. The reactor was sealed and heated while the contents were agitated. The temperatures and pressures at various times after heating was begun are shown in Table 1.

TABLE 1

| Time, Hours:Minutes | Temperature, °C | Pressure, Kilopascals Gauge |
|---|---|---|
| 0:0 | Room | 0 |
| 0:10 | 55 | 138 |
| 0:50 | 160 | 827 |
| 1:20 | 180 | 1172 |
| 2:40 | 180 | 1241 |
| 3:50 | 180 | 1379 |
| 4:25 | 180 | 1448 |
| 4:50 | 180 | 1517 |

Four hours and fifty minutes after heating was begun, heating was discontinued and the reactor was allowed to cool. Twenty-three hours and thirty-five minutes after heating was begun, the temperature and pressure were observed to be room temperature and 207 kilopascals gauge, respectively. A sample of gas taken from the reactor was analyzed and found to contain carbon dioxide. The pressure was reduced to ambient and the reactor was found to contain 198.0 grams of liquid. After removal of dimethyl carbonate and methanol by vacuum stripping, analysis of the solidified residue by liquid chromatography showed it to contain 63 area percent N,N-bis(2,4,6-tribromophenyl)methylamine and 36 area percent bis(2,4,6-tribromophenyl)amine.

EXAMPLE II

The reactor of Example I was charged with 50 grams of bis(2,4,6-tribromophenyl)amine and 160 grams of dimethyl carbonate. The reactor was sealed and heated while the contents were agitated. The temperatures and pressures at various times after heating was begun are shown in Table 2.

TABLE 2

| Time, Hours:Minutes | Temperature, °C | Pressure, Kilopascals Gauge |
|---|---|---|
| 0:0 | Room | 0 |
| 0:35 | 185 | 1517 |
| 1:35 | 195 | 1724 |
| 2:43 | 195 | 2068 |
| 3:05 | 195 | 2206 |
| 4:05 | 195 | 2413 |
| 4:50 | 195 | 2482 |

Four hours and fifty minutes after heating was begun, heating was discontinued and the reactor was allowed to cool. Twenty-three hours and twenty minutes after heating was begun, the temperature and pressure were observed to be room temperature and 276 kilopascals gauge, respectively. The pressure was reduced to ambient and the reactor was found to contain 195 grams of liquid. After removal of dimethyl carbonate and methanol by vacuum stripping, analysis of the solidified residue liquid chromatography

showed it to contain 87 area percent N,N-bis(2,4,6-tribromophenyl)methylamine and 11 area percent bis(2,4,6-tribromophenyl)amine. N,N-bis(2,4,6-tribromophenyl)methylamine is useful as a fire retardant additive for polymers, especially, acrylonitrile-butadienestyrene interpolymers and high density polyethylene.

Although the present invention has been described with reference to specific details of certain embodiments thereof, it is not intended that such details should be regarded as limitations upon the scope of the invention except insofar as they are included in the accompanying claims.

**Claims**

1. N,N-Bis(2,4,6-tribromophenyl)methylamine.
2. A method of preparing the compound of claim 1 by reacting Bis(2,4,6-tribromophenyl)amine with a dialkyl carbonate.

**Patentansprüche**

1. N,N-Bis(2,4,6-tribromophenyl)methylamin.
2. Verfahren zum Herstellen der Verbindung von Anspruch 1 durch Umsetzen von Bis(2,4,6-tribromophényl)amin mit einem Dialkylcarbonat.

**Revendications**

1. N,N-Bis(2,4,6-tribromophényl)méthylamine.
2. Procédé de préparation du composé de la revendication 1 par réaction de la bis(2,4,6-tribromophényl)amine avec un carbonate de dialkyle.